(19) Europäisches Patentamt / European Patent Office / Office européen des brevets

(11) **EP 4 093 494 B1**

(12) **EUROPEAN PATENT SPECIFICATION**

(45) Date of publication and mention
of the grant of the patent:
**13.09.2023 Bulletin 2023/37**

(21) Application number: **21702066.8**

(22) Date of filing: **20.01.2021**

(51) International Patent Classification (IPC):
*A61N 1/36* (2006.01)    *A61N 1/04* (2006.01)

(52) Cooperative Patent Classification (CPC):
**A61N 1/3603; A61N 1/0456; A61N 1/36025**

(86) International application number:
**PCT/GB2021/050123**

(87) International publication number:
**WO 2021/148787 (29.07.2021 Gazette 2021/30)**

(54) **METHOD FOR OBTAINING PERSONALIZED PARAMETERS FOR TRANSCRANIAL STIMULATION, TRANSCRANIAL STIMULATION SYSTEM**

**VERFAHREN ZUR GEWINNUNG VON PERSONALISIERTEN PARAMETERN FÜR TRANSKRANIELLE STIMULATION, TRANSKRANIELLES STIMULATIONSSYSTEM**

**PROCÉDÉ D'OBTENTION DE PARAMÈTRES PERSONNALISÉS RELATIFS À LA STIMULATION TRANSCRÂNIENNE, SYSTÈME DE STIMULATION TRANSCRÂNIENNE**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.01.2020 GB 202000874**

(43) Date of publication of application:
**30.11.2022 Bulletin 2022/48**

(73) Proprietor: **The University of Surrey**
**University Campus**
**Guildford**
**Surrey GU2 7XH (GB)**

(72) Inventors:
- **COHEN KADOSH, Roi**
  **Oxford Oxfordshire OX2 9AQ (GB)**
- **REED, Thomas**
  **Salisbury Wiltshire SP5 3SB (GB)**
- **NGUYEN, Vu**
  **Oxford Oxfordshire OX5 1ND (GB)**
- **VAN BUEREN, Nienke**
  **3621 XZ Breukelen (NL)**

(74) Representative: **Whiting, Gary**
**Venner Shipley LLP**
**5 Stirling House**
**Stirling Road**
**The Surrey Research Park**
**Guildford GU2 7RF (GB)**

(56) References cited:
**WO-A1-2019/152858    US-A1- 2016 175 586
US-A1- 2019 321 583**

- **LORENZ ROMY ET AL: "Efficiently searching through large tACS parameter spaces using closed-loop Bayesian optimization", BRAIN STIMULATION, ELSEVIER, AMSTERDAM, NL, vol. 12, no. 6, 4 July 2019 (2019-07-04), pages 1484-1489, XP085897140, ISSN: 1935-861X, DOI: 10.1016/J.BRS.2019.07.003 [retrieved on 2019-07-04]**
- **DESAUTELS THOMAS A ET AL: "An Active Learning Algorithm for Control of Epidural Electrostimulation", IEEE TRANSACTIONS ON BIOMEDICAL ENGINEERING, IEEE SERVICE CENTER, PISCATAWAY, NJ, USA, vol. 62, no. 10, 1 October 2015 (2015-10-01), pages 2443-2455, XP011668910, ISSN: 0018-9294, DOI: 10.1109/TBME.2015.2431911 [retrieved on 2015-09-16]**

**Description**

The invention is defined in the appended claims. Aspects, embodiments and examples disclosed herein which do not fall within the scope of the appended claims do not form part of the invention, and are merely provided for illustrative purposes,

The invention relates to transcranial stimulation.

It is known in the art that transcranial stimulation can be used to treat patients or to achieve cognitive enhancement in healthy individuals. For example, US2019/0321583 discloses a method of replicating a mental state of a first subject in a second subject by inducing the brain activity patterns of the first subject in the second subject. Optimal parameters for the stimulation may differ between different individuals but need to be selected from a large range of possible combinations. As a consequence, a practical approach has been to use a one-size-fits-all methodology in which the same set of parameters are used for multiple different subjects.

It is an object of the invention to improve transcranial stimulation.

According to one aspect of the invention, there is provided a computer-implemented method for obtaining personalized parameters for transcranial stimulation, comprising: receiving baseline data about a test subject, the baseline data comprising information about the test subject acquired prior to any transcranial stimulation applied to the test subject; and using a Gaussian process model of performance of one or more training subjects to obtain personalized parameters for transcranial stimulation for the test subject based on the received baseline data, wherein: the Gaussian process jointly models subject performance during and/or after transcranial stimulation as a function of both i) parameters defining the transcranial stimulation; and ii) baseline data for the one or more training subjects.

Thus, a probabilistic model is provided that intrinsically takes account of variations between different people by means of baseline data. By taking account of the baseline data in this way the inventors have found that the model is better able to predict optimal parameters for the transcranial stimulation even where past data from no or very few subjects with the same or similar baseline data is available. The method is able to provide personalized parameters that vary between different subjects and, on average, perform better than a one-size-fits-all methodology which ignores the fact that people differ from each other, and that the individual brain is plastic and changes over time as well. The present method makes it possible to effectively tailor the stimulation protocol to provide the best outcome. The stimulation may be used for various clinical and non-clinical purposes, including for example improving tremor in Parkinson's, improving people's sustained attention/concentration, memory, mathematical performance, emotions, training capabilities, and learning (e.g., language, maths, IT, factual information).

In an embodiment, the baseline data represents a performance (e.g. cognitive performance and/or motor performance, subjective report) without influence from transcranial stimulation. The inventors have found that using baseline data of this type makes it possible to obtain personalized parameters particularly effectively.

In an embodiment, the obtaining of the personalized parameters further comprises: obtaining test data representing performances (e.g. cognitive performance and/or motor performance, subjective report) of the test subject during and/or after application of transcranial stimulation with multiple respective combinations of parameters; refining the Gaussian process model using the obtained test data; and using the refined Gaussian process model to obtain the personalized parameters. In an embodiment, the Gaussian process model is refined in an iterative Bayesian optimization process that in each step chooses to sample next where a combination of parameters for stimulation optimizes an acquisition function. Using an acquisition function in this way allows the Gaussian process model to explore the most relevant parts of the available parameter space in an efficient manner, thereby promoting rapid and reliable convergence to optimal personalized parameters for the test subject.

Embodiments of the invention will now be described, by way of example only, with reference to the accompanying drawings in which corresponding reference symbols indicate corresponding parts, and in which:

Figure 1 is a flow chart schematically depicting a method for obtaining personalized parameters for transcranial stimulation;

Figures 2-5 schematically depict a Bayesian optimization process;

Figures 6-8 depict maps of predicted cognitive performance during stimulation of subjects having three different baseline cognitive performances, as a function of frequency and current of applied stimulation;

Figure 9 is a graph showing variation of an optimized performance of subjects as a function of baseline cognitive performance;

Figure 10 is a graph showing variation of personalized parameters (frequency (broken line) and current (solid line)) as a function of baseline cognitive performance;

Figure 11 depicts optimisation of a black-box function f(x) as a function of number of iterations in adjustment of a Gaussian process model;

Figure 12 is a graph showing results of a simulation analysis comparing the performance of embodiments of the present disclosure (upper curve) with performance based on randomly sampling a parameter space;

Figure 13 is a graph comparing use of Bayesian optimization that only models parameters for stimulation (lower curve) with personalized Bayesian optimization that jointly models both parameters for stimulation and baseline data for subjects (upper curve);

Figure 14 schematically depicts a transcranial stimulation system;

Figure 15 is a graph depicting a 3D visualization of a simulated function for demonstrating use of a method of obtaining personalized parameters for transcranial electrical stimulation for use in treating Attention Deficit Hyperactivity Disorder (ADHD);

Figure 16 is a graph depicted a 2D visualization of the simulated function shown in Figure 15;

Figure 17 is a graph depicting a 2D visualization of the simulated function of Figure 16 with true optimal parameters plotted as black dots for each Neurophysiology value (the true optimal parameters are unknown to the scientist/clinician);

Figure 18 is the graph of Figure 17 with suggested parameters from different algorithms overlayed (parameters suggested by personalized Bayesian optimization are shown by white squares and parameters suggested by Bayesian optimization are shown by a white broken line);

Figure 19 is a graph depicting a 2D visualization of the simulated function with selected parameters from personalized Bayesian optimization plotted over iterations. The randomly chosen points from the initial phase are displayed in black and the points selected by personalized Bayesian optimization are shown in white; and

Figure 20 is a graph comparing the performance across different methods.

Transcranial alternating current stimulation (tACS) and similar techniques are believed to be able to promote oscillatory activity via transcranial stimulation. Such techniques may modulate brain oscillations that subserve cognitive processes. It is possible to enhance or disrupt the effects of the stimulation by changing parameters of the stimulation (e.g., current and frequency), for example to target a specific neural population. However, exploring the effects of every combination of parameters individually on a test subject (e.g., on cognitive performance) is not practical. It has therefore been difficult to use transcranial stimulation techniques such as tACS optimally. Embodiments of the present disclosure address this issue.

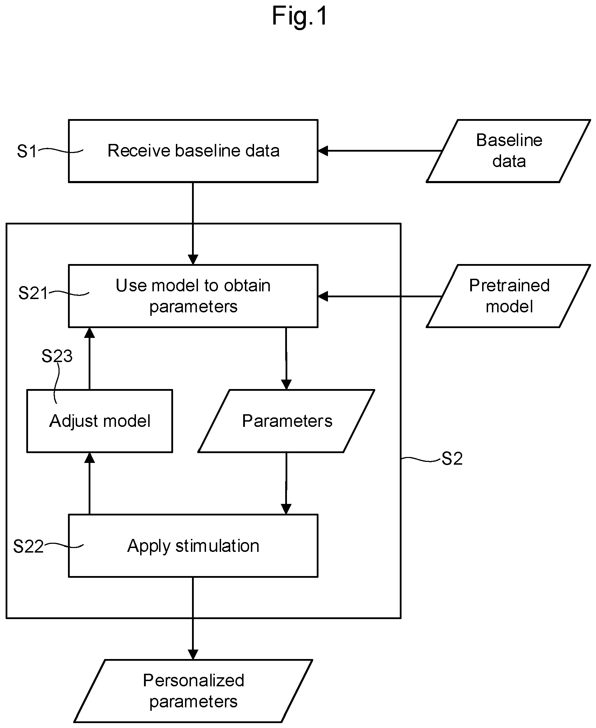

Figure 1 schematically depicts a framework for methods of obtaining personalized parameters for transcranial stimulation. The methods may be computer-implemented. Each of one or more of the steps of each method may therefore be performed by a computer. The computer may comprise various combinations of computer hardware, including for example CPUs, RAM, SSDs, motherboards, network connections, firmware, software, and/or other elements known in the art that allow the computer hardware to perform the required computing operations. The required computing operations may be defined by one or more computer programs. The one or more computer programs may be provided in the form of media or data carriers, optionally non-transitory media, storing computer readable instructions. When the computer readable instructions are read by the computer, the computer performs the required method steps. The computer may consist of a self-contained unit, such as a general-purpose desktop computer, laptop, tablet, mobile telephone, or other smart device. Alternatively, the computer may consist of a distributed computing system having plural different computers connected to each other via a network such as the internet or an intranet. The methods may thus be performed partly or entirely by cloud computing facilities.

In step S1 of Figure 1, the method comprises receiving baseline data about a test subject. The baseline data may be received from a data storage device or directly from a device that measures the baseline data, or based on a subjective report. A subject report may be done by the subject (e.g. reporting how tired he/she feels) or by a third party (e.g. reporting how distracted a subject is). In an embodiment, the baseline data comprises information about the test subject that is acquired prior to (and/or separately from and/or independently of) transcranial stimulation applied to the test subject. In an embodiment, the baseline data represents a performance (e.g. cognitive performance and/or motor performance, subjective report) of the test subject without influence from transcranial stimulation. The baseline data may thus be obtained during a time period when transcranial stimulation is not being applied to the subject and/or has not been applied so recently that the subject's performance is significantly influenced by the stimulation. In some embodiments, the baseline data alternatively or additionally comprises other personal information about the subject, such as neural data and/or demographic data (e.g., information about sleep patterns, lifestyle habits such as smoking, alcohol consumption, age, gender, etc.).

Performance may be measured in various ways, providing a corresponding variety of possibilities for a format of the baseline data (in embodiments where the baseline data comprises information about performance). For example, measurements of accuracy and/or reaction times during tests may be made. In some embodiments, the baseline data comprises a scalar numerical value that may optionally vary continuously as a function of cognitive performance (e.g., a test score or IQ score). In some embodiments, the baseline data comprises a drift rate value obtained by applying a diffusion decision model to the results of tests performed by the subject. The tests may comprise any suitable activity that provides information about performance. In some embodiments, as exemplified below, the tests comprise mathematical tests, such as arithmetic tests. In some embodiments, arithmetic tests comprising single-digit times multi-digit multiplications are used. Modelling human behaviour using a diffusion decision model facilitates pinpointing of cognitive processes of interest in preference to more peripheral processes such as stimulus encoding or motor-related activity. This approach makes it possible to dissect different components in the chain of information processing by modelling the decision process and targeting the component that reflects ability/task difficulty (e.g., the drift rate, which combines response time and accuracy), rather than auxiliary components. Additionally, the approach allows simultaneous characterisation of changes in accuracy and reaction time in the form of a single metric value.

Skills needed for solving arithmetic problems vary greatly, not only between people with learning deficits but also in the general population. Similarly, a recent study highlighted the importance of individual differences in both neural and behavioural correlates that differ between people with high and low arithmetic skills. The left frontoparietal network has been implicated to play an important role in arithmetic processing and can be easily targeted by transcranial stimulation techniques such as tACS. A recent tACS study indicated the importance of this network in the left hemisphere in the memory domain which is also thought to underly stepwise calculation of arithmetic problems. The present method can improve targeting of this network by providing personalized parameters for the stimulation.

In step S2 of Figure 1, the method comprises using a Gaussian process model of performance (e.g. cognitive performance and/or motor performance, subjective report) of one or more training subjects to obtain personalized parameters for transcranial stimulation for the test subject based on the baseline data received in step S1. The Gaussian process model jointly models performance (cognitive performance and/or motor performance, subjective report) during and/or after transcranial stimulation as a function of both: 1) parameters defining the transcranial stimulation; and 2) baseline data for the one or more training subjects.

In an embodiment, the obtaining of the personalized parameters comprises an iterative refining of the Gaussian process model using measurements of performance (e.g. cognitive and/or motor performance, subjective report) of the test subject during application of transcranial stimulation with multiple combinations of parameters. In some embodiments, the parameters include one or more of the following: frequency, frequencies, current, phase, duration, dose, brain region. Different combinations of currents in the range 0.4-2 mA and frequencies in the range of 0.1-100 Hz may be considered, for example. Where the stimulation comprises waveforms that are more complex than pure sinusoids, other parameters may be included to define relevant additional features of the waveforms (e.g., to define aspects of the frequency spectrum of the waveforms such as central frequency and bandwidth, band shape, pulse length, etc.). In the example procedure depicted in Figure 1, the iterative refinement of the model comprises iterative performance of sub-steps S21, S22 and S23. In some embodiments, the obtaining of the personalized parameters may include selecting between different overall modes of stimulation (e.g. between tACS, transcranial direct current stimulation (tDCS), transcranial random noise stimulation (tRNS) and/or other modes of stimulation), either during treatment of an individual or between different individuals.

In the example shown, in an initial instance of sub-step S21, initial parameters for stimulation for the test subject are obtained using only the pretrained Gaussian process model and the baseline data. In this embodiment, the initial operating parameters are thus obtained without yet having performed any measurements of performance (e.g. cognitive performance and/or motor performance, subjective report) during and/or after application of transcranial stimulation to the test subject. The pretrained Gaussian process model may be trained on range of previously collected data. This data can come from the random assignment of stimulation parameters. Technically the Gaussian process (GP) does not explore the different combinations, it just models the existing data and provides estimates of the response to the variety of stimulation parameters as well as an estimate of uncertainty. The exploration is done by the acquisition function which is the next stage of the Bayesian optimisation process.

In subsequent sub-step S22, a performance (e.g. cognitive performance and/or motor performance, subjective report) of the test subject during and/or after transcranial stimulation is measured. The transcranial stimulation is applied using the parameters provided by sub-step S21. The measured performance forms test data. During iteration, sub-step 22 is repeated, thereby obtaining test data that represents measurements of performance during and/or after application of transcranial stimulation with multiple respective combinations of operating parameters (e.g., the operating parameters have different combinations of values during each iteration).

In subsequent sub-step S23, the test data obtained in sub-step S22 is used to adjust the Gaussian process model that was used in the preceding instance of sub-step S21. Sub-steps S21, S22 and S23 are then iteratively repeated until the measured performance (e.g. cognitive performance and/or motor performance, subjective report) obtained in sub-step S22 converges to an optimal value (e.g., when a rate of improvement falls below a predetermined threshold value) and/or a predetermined number of iterations has been performed. Thus, the Gaussian process model is refined using the obtained test data (obtained over multiple iterations of sub-steps S21, S22 and S23 in the example shown). This optimization process may be performed across multiple different users (with each user using the process at least once) and/or multiple uses by each of one or more individual users.

The refined Gaussian process model at convergence provides a prediction of how subject performance (e.g. cognitive performance and/or motor performance, subjective report) is expected to vary as a function of parameters for stimulation for the test subject. The combination of parameters that are predicted by the refined Gaussian process model to give the maximum performance may thus be output as the personalized parameters for the test subject. The refined Gaussian process model is thus used to obtain the personalized parameters.

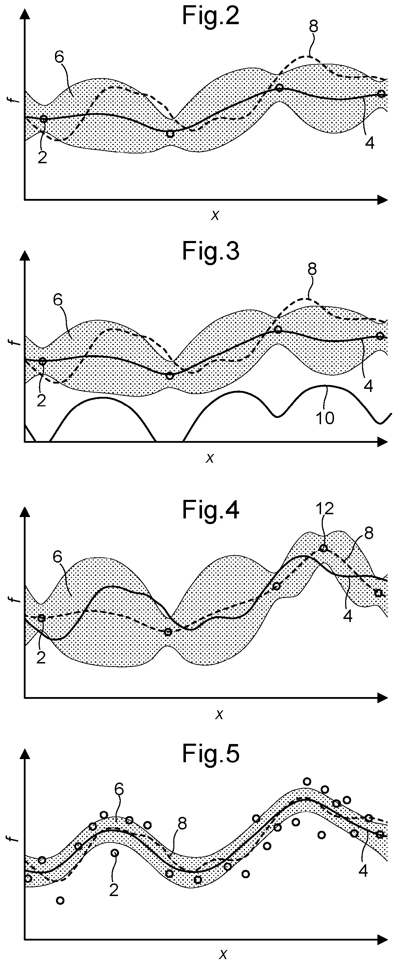

The Gaussian process model may be refined in an iterative Bayesian optimization (BO) process, as depicted schematically in Figures 2-5. BO is an active machine learning technique that aims to find the global optimum of a black-box function $f(x)$ by making a series of evaluations. In Figures 2-5, the vertical axes represent $f(x)$ and the horizontal axes represent $x$ (in a schematic one-dimensional form for ease of illustration, but it will be understood that $x$ will be multidimensional in embodiments of the present disclosure, including for example one dimension for each type of parameter for simulation, such as current and frequency, and one dimension for baseline data).

As depicted in Figure 2, a BO process may initially comprise fitting a Gaussian process model to available data points (a representative point is labelled "2" in Figures 2-5). The fitted Gaussian process model has an associated estimated mean (labelled "4") and uncertainty (labelled "6"). An example objective function is labelled "8".

The Gaussian process model can be refined by providing further data points. In methods of the present disclosure, the further data points may comprise measurements of performance (e.g. cognitive performance and/or motor performance, subjective report) of the test subject during and/or after application of transcranial stimulation with different combinations of parameters, such as is provided by the test data generated during each iteration of the sub-step S22 in Figure 1, or test data provided from other test subjects.

In each step the iterative BO process may be configured to choose to sample next where $x$ optimizes (e.g., maximizes) an acquisition function 10, as depicted in Figure 3. In embodiments of the present disclosure, the BO process may thus choose to sample next where a combination of parameters for stimulation optimizes (e.g., maximizes) an acquisition function 10. The acquisition function 10 is derived based on the Gaussian process model but is configured to be computationally cheaper to optimize than the objective function of the Gaussian process model. The acquisition function may be chosen to promote steering of sampling in the direction where improvement over the current best evaluation (e.g., highest cognitive performance) is most likely. The nature of the acquisition function is not particularly limited. The acquisition function may, for example, comprise a known acquisition function such as the Gaussian process upper confidence bound (GP-UCB) and/or the expected improvement (EI). Further example details about the acquisition function are given below.

As depicted in Figure 4, a value of $x$ (e.g., corresponding to a particular combination of parameters for stimulation) at an optimal point (a maximum in the example shown) of the acquisition function 10 shown in Figure 3 is selected as the next data point (labelled "12") to sample in the BO process. The Gaussian process model is adjusted (e.g., by adjusting the hyperparameters) to take account of the new data point 12, resulting in a new estimated mean 4 and uncertainty 6. The BO process is continued by progressively adding further data points until a potential improvement is considered negligible (e.g., convergence). Figure 5 schematically depicts a state of convergence in which the Gaussian process more accurately models the objective function 4 with a relatively narrow band of uncertainty. In embodiments of the present disclosure, parameters for stimulation corresponding to a maximum of the estimated mean performance in the converged Gaussian process model may be output as the personalized parameters for the test subject.

The procedure described above for iteratively refining a Gaussian process model may also be used for providing the Gaussian process model that is initially input to step S21 in Figure 1. The training of the Gaussian process model may, for example, use training data representing performances (e.g. cognitive performances and/or motor performances, subjective report) of each of multiple training subjects during and/or after application of transcranial stimulation with multiple respective combinations of parameters for each training subject. As described above, the Gaussian process model may be trained in an iterative Bayesian optimization process that in each step chooses to sample next where a combination of parameters for stimulation optimizes (e.g., maximizes) an acquisition function.

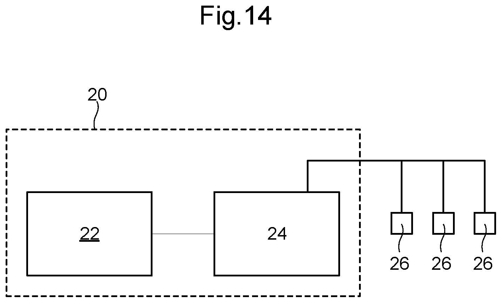

As depicted schematically in Figure 14, a data processing apparatus 22 may be provided for performing any of one or more of the computer-implemented methods described above. The data processing apparatus 22 may be provided as a stand-alone device or may be incorporated into a transcranial stimulation system 20, as depicted in Figure 14. The transcranial stimulation system 20 may comprise a stimulation unit 24 that provides a stimulation to a subject. The transcranial stimulation may comprise any one or more of the following: transcranial alternating current stimulation; transcranial random noise stimulation; transcranial direct current stimulation; transcranial magnetic stimulation; transcranial focused ultrasound; and transcranial light stimulation. The stimulation unit 24 comprises suitable hardware for applying the relevant stimulation (e.g., one or more electrodes 26 for transcranial electrical stimulation in the example shown). The data processing apparatus 20 controls operating parameters of the stimulation unit 24 to define parameters for stimulation. The parameters for stimulation may comprise one or more of the following: frequency, frequencies, current, phase, duration, dose, and brain region. The data processing apparatus 20 may perform any of the computer-implemented methods described above that provide personalized parameters for the subject and control the operating parameters of the stimulation unit 24 based on the obtained personalized parameters for the subject.

DETAILED EXAMPLE

*Further details about Bayesian optimisation*

A function *f* may be used to denote an unknown black-box function for which we do not have a closed-form expression, but we could have an infinite number of queries. Furthermore, this black-box function is expensive and time costly to evaluate. Formally, let $f : X \to \mathbb{R}$ (a set of real numbers representing the values from -∞ to +∞) be a well-behaved function defined on a subset $X \subseteq \mathbb{R}^d$ whereby *d* is the number of dimensions. In the case where *f* represents cognitive performance, solving the following global optimisation problem could provide improved optimal parameters x*for applying transcranial stimulation

$$x^* = \underset{x \in X}{\operatorname{argmax}} f(\boldsymbol{x})$$

The BO algorithm can be used for example to find the global optimum of arithmetic performance, as indicated by drift rates, of the black-box function *f(**x**)* by making a series of evaluations $x_1$, $x_2$, ..., $x_T$. This approach does not, however, yet take optimal account of variations in baseline ability (e.g., baseline cognitive performance) between different subjects.

*Taking account of different baseline data (e.g., cognitive performances)*

Each subject has individual baseline data, representing for example a baseline cognitive performance (e.g., arithmetic ability), which may be represented by a baseline data value *c*. The baseline data value *c* is provided separately for every subject. The inventors have found that optimal parameters to use for transcranial stimulation vary significantly between subjects with different baselines. That is, the optimal parameter *x** is not unique for different baseline data values *c*. The inventors have recognised that methods of obtaining parameters for stimulation can be improved by adapting the global optimisation problem described above to intrinsically take account of the baseline data.

Taking account of the baseline data can be done by recasting the global optimization problem so as to be defined formally as:

$$x^*(c) = \underset{x \in X}{\operatorname{argmax}} f(\boldsymbol{x}, c)$$

where *c* is the baseline data value. The optimal parameter *x** is not defined globally, but specifically to the variable *c*.

*Gaussian process for joint modelling of parameters for stimulation and baseline data*

The traditional BO (without considering baseline data values c) reasons about *f* by building a Gaussian process (GP) through evaluations *f ~GP(m,k)*, where *m* is the prior mean function and *k* is the covariance function. This flexible distribution allows us to associate a normally distributed random variable at every point in the continuous input space. Therefore, we get the predictive distribution for a new observation that also follows a Gaussian distribution and its mean ($\mu$) and variance ($\sigma^2$) are given by:

$$\mu(\boldsymbol{x}') = \boldsymbol{k}(\boldsymbol{x}'; X) \boldsymbol{K}(X; X)^{-1} \boldsymbol{y}$$

$$\sigma^2(\boldsymbol{x}') = k(\boldsymbol{x}'; \boldsymbol{x}') - \boldsymbol{k}(\boldsymbol{x}'; X) \boldsymbol{K}(X; X)^{-1} \boldsymbol{k}(\boldsymbol{x}'; X)^T$$

where *K(U; V)* is a covariance matrix whose element *(i;j)* is calculated as $k_{i,j} = k(\boldsymbol{x}_i; \boldsymbol{x}_j)$ with $\boldsymbol{x}_i \in U$ and $\boldsymbol{x}_j \in V$. Behavioural observations are typically associated with noise that can be accommodated in a Gaussian process model. Namely, every *f(**x**)* has an extra covariance with itself since the noise is assumed to be independent including a magnitude equal to the noise variance:

$y_i = f(\boldsymbol{x}_i) + \varepsilon_i$ where $\epsilon_i \sim N(0, \sigma_n^2)$ where $\sigma_n^2$ is the noise variance.

When considering noise, the output follows the Gaussian process as $y \sim GP(m, k + \sigma_n^2 \delta_{i,j})$ where $\delta_{i,j}$ = 1 if $i = j$ is the Kronecker's delta. The covariance function for a noisy process becomes the sum of the signal covariance and the noise covariance. Particularly, the covariance function between two observations can be computed as:

$$k\left(\boldsymbol{x}_i; \boldsymbol{x}_j\right) = \exp(-\frac{(\boldsymbol{x}_i - \boldsymbol{x}_j)^2}{2\sigma_l^2}) + \sigma_n^2 \delta_{i,j}$$

To take account of the baseline data values $c$, one possible approach would be to build a Gaussian process and optimisation for each baseline data value $c$, respectively. However, such an approach suffers a critical problem of data efficiency. To elaborate, the number of data samples is not sufficient to estimate each baseline data value $c$ separately. In embodiments of the present disclosure, this shortcoming is overcome by extending the Gaussian process surrogate to jointly model our target function $f$ and the additional baseline data value dimension c. In this case, the GP covariance becomes:

$$k\left(\{\boldsymbol{x}_i, c_i\}; \{\boldsymbol{x}_j, c_j\}\right) = k(\boldsymbol{x}_i, \boldsymbol{x}_j) \times k(c_i, c_j)$$

where $k(\boldsymbol{x}_i, \boldsymbol{x}_j)$ is defined above and $k\left(c_i, c_j\right) = \exp(-\frac{(c_i - c_j)^2}{2\sigma_c^2})$ . These covariance functions correspond to the parameters for stimulation and baseline data values respectively. The length scale parameter $\sigma_c^2$ used in $k(c_i, c_j)$ is different from $\sigma_x^2$ used in $k(\boldsymbol{x}_i, \boldsymbol{x}_j)$. For example, if the baseline data length-scale $\sigma_c^2$ is extremely large, it means the performance function is not changing with respect to the baseline data (e.g., baseline performance). On the other hand, if $\sigma_c^2$ is small, it means the performance function is changing rapidly with the baseline data. We later use the maximum marginal likelihood to estimate these length scale parameters from the data directly. Under our modification for the GP to take account of baseline data values $c$, we can estimate the predictive mean and predictive variance as follows:

$$\mu(\boldsymbol{x}, c) = \boldsymbol{k}(\{\boldsymbol{x}, c\}; Z)\boldsymbol{K}(Z; Z)^{-1}\boldsymbol{y}$$

$$\sigma^2(\boldsymbol{x}, c) = k(\{\boldsymbol{x}, c\}; \{\boldsymbol{x}, c\}) - \boldsymbol{k}(\{\boldsymbol{x}, c\}; Z)\boldsymbol{K}(Z; Z)^{-1}\boldsymbol{k}(\{\boldsymbol{x}, c\}; Z)^T$$

where we denote $Z = [X, C]$, and the contextualised covariance matrix $k$ is defined above.

*Acquisition Function*

To select the next point to evaluate, the acquisition function $\alpha(\boldsymbol{x})$ will be chosen to construct a utility function that is based on the Gaussian process model mentioned above. Instead of maximizing the expensive original function $f$, we maximize the cheaper acquisition function to select the next most optimum point:

$$x_{t+1} = \underset{x \in X}{\operatorname{argmax}} \alpha(\boldsymbol{x})$$

In this auxiliary maximisation problem, the acquisition function form is known and can be easily optimised by standard numerical techniques. One option for the acquisition function is the Gaussian process upper confidence bound (GP-UCB):

$$\alpha(\boldsymbol{x}, c) = \mu(\boldsymbol{x}, c) + \kappa \times \sigma(\boldsymbol{x}, c)$$

where $\mu(\boldsymbol{x},c)$ and $\sigma(\boldsymbol{x},c)$ are the GP predictive mean and variance defined above. In the above formula, $\kappa$ is the parameter controlling the exploration and exploitation tradeoff. The value of $\kappa$ can be theoretically chosen following the approach presented in Srinivas, Niranjan, Andreas Krause, Sham Kakade, and Matthias Seeger. "Gaussian process optimization in the bandit setting: no regret and experimental design." In Proceedings of the 27th International Conference on International Conference on Machine Learning, pp. 1015-1022. 2010.

In examples performed in the present study, the following acquisition function $\alpha(\boldsymbol{x})$ was used:

$$\alpha(\boldsymbol{x}) = \frac{Vstim}{Vbase}$$

where *Vstim* is the drift rate during the 50 trials of an arithmetic multiplication block that is normalized by the *Vbase* which is the drift rate calculated over 25 trials from the baseline task. To determine improvement between the baseline and the stimulation block, a second Vbase was calculated over the other 25 trials from the baseline task. A Pearson correlation was calculated to determine if the two drift rates used as improvement index did not differ (r=.57, p<.001). Acquisition functions were designed to trade-off between exploration of the search space and exploitation of current promising regions. A burn-in phase of 60 random tACS frequency-current combinations was used that were assigned to the first 20 participants of the BO design to determine the amount of variation induced by stimulation. We decided to use a large burn-in in our paradigm to reliably design a Bayesian Optimisation algorithm that is based on a high amount of data.

To pick the hyperparameters (estimated parameters without using observed data) of the next stimulation block, one can optimize the expected improvement (EI) over the current best result or the GP-UCB. In short, it is more likely that the UCB selects evaluations with both a high mean and high variance. EI and UCB have been shown to be efficient in the number of function evaluations required to find the global optimum of many multimodal black-box functions. During the present study, the EI was applied to find the optimum in arithmetic performance. Lastly, we decided to remove one drift rate value of 3.6 during the experimental procedure due to possible ceiling effects the BO. In addition, due to technical problems one data point was not included in the BO procedure. In total, we acquired 148 iterations.

RESULTS

*Group-level baseline personalized Bayesian optimisation*

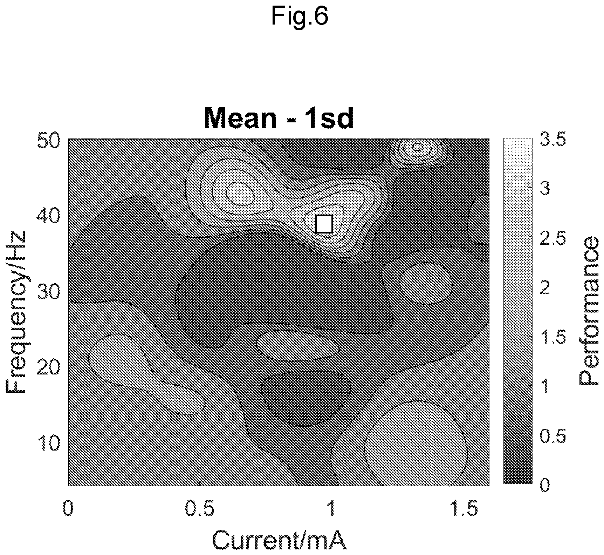

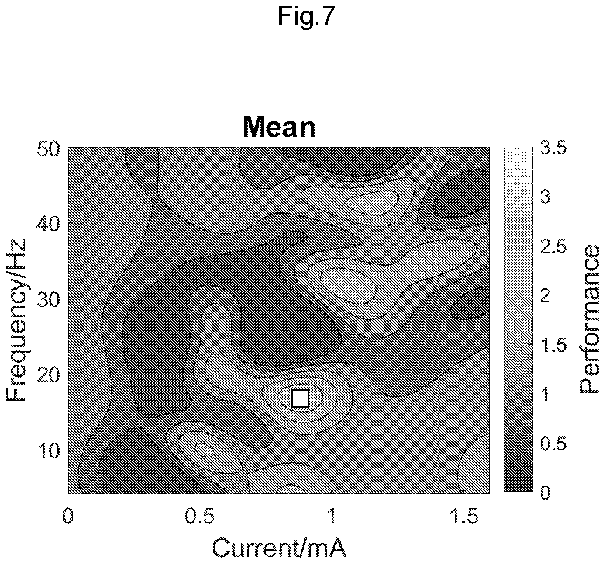

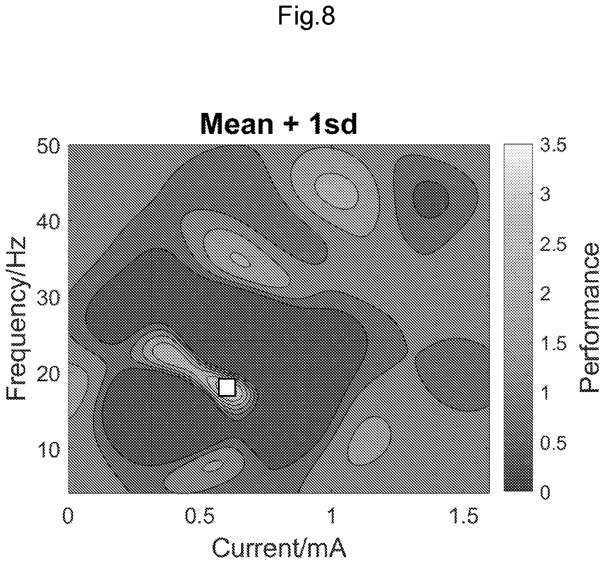

Figures 6-8 depict maps of predicted cognitive performance during stimulation of three respective groups of subjects having different baseline cognitive performances, as a function of frequency (vertical axis) and current (horizontal axis) of applied stimulation. The predicted cognitive performance may be obtained using a Gaussian process model that jointly models performance as a function of parameters for stimulation and baseline data values *c*, as described above with reference to Figures 1-5. Figure 6 depicts a model for relatively poor baseline performance (1SD below a mean drift rate). Figure 7 depicts a model for mean baseline performance (mean=0.055). Figure 8 depicts a model for relatively high baseline performance (1SD above the mean drift rate). The vertical axis shows the frequency range of the applied stimulation (0-50 Hz) and the horizontal axis shows the current of the stimulation (0-1.6 mA). Arithmetic performance is indicated by shading based on the calculated drift rates (tACS block/baseline block). Low drift rates are shown in darker shading and high drift rates are shown in lighter shading. A best inferred point for arithmetic performance, which can be used as the basis to provide optimal personalized parameters for stimulation, is indicated by a white square in each figure.

Figures 6-8 show that there is a marked shift from higher to lower frequencies and currents from lower (poor) to higher (strong) baseline performances: for the mean-1SD example of Figure 6, the optimal operating parameters were 38.67Hz and 0.97mA peak-to-peak; for the mean example of Figure 7, the optimal operating parameters were 16.67Hz and 0.88mA peak-to-peak, and for the mean+1SD example, the optimal operating parameters were 18Hz and 0.60mA peak-to-peak. In this example, the baseline data value was a continuous parameter, such that the best inferred combination of parameters is expected to vary along this continuum. It should also be noted that the results of Figures 6-8 are derived from tests that included a larger number of subjects with lower than average baseline data values (representing lower than average baseline cognitive performances) than subjects with higher than average baseline data values. Therefore, the results of Figure 6 are of higher confidence regarding an estimation of the optimum frequency-current parameter combination than the results of Figure 8.

*Validation of optimising arithmetic performance by Bayesian optimisation*

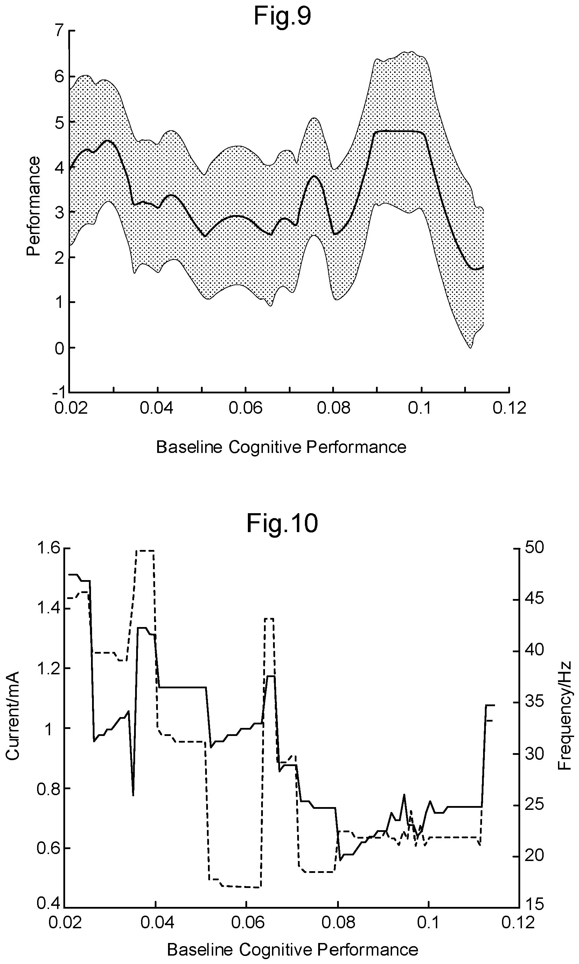

Figure 9 is a graph showing variation of an optimized performance (middle curve) of subjects as a function of baseline cognitive performance. 95% confidence intervals are indicated by shading. Figure 9 shows a fluctuation across subjects with low and high baseline data values but a significant improvement is seen for all subjects, with the stimulation not being markedly more effective for any particular group of subjects (range of baseline data values).

Figure 10 depicts the variation in personalized tACS parameters (vertical axes) proposed by the BO algorithm as a function of baseline cognitive performance (horizontal axis). The solid line represents variation of stimulation current with baseline cognitive performance and the broken line represents variation of stimulation frequency with baseline cognitive performance. This plot confirms a shift from higher frequencies and currents in low baseline ability subjects to lower frequencies and currents when baseline ability increases, consistent with the group-level BO models shown in Figures 6-8.

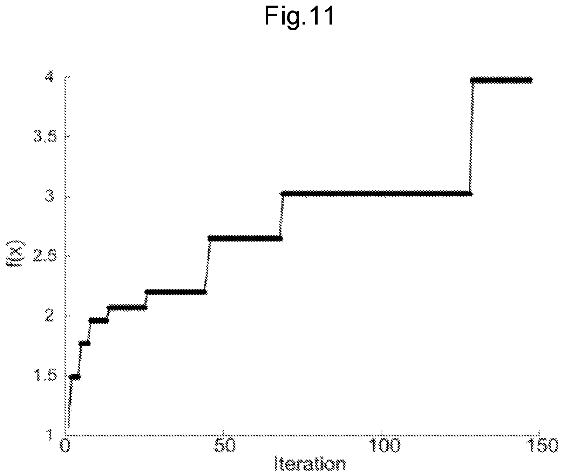

Figure 11 depicts increased optimisation of the black-box function $f(x)$ with EI as acquisition function along an increase in number of iterations. The stable plateauing shows that the black-box function $f(x)$ is reliably optimised after every iteration.

*Personalized Bayesian optimisation*

To the inventors' knowledge, the current study is the first to attempt to optimise complex human behaviour by means of a personalized BO approach to obtaining parameters for transcranial stimulation, with the BO being configured to jointly model parameters for stimulation and baseline data representing personal information about a subject that is independent of any transcranial stimulation, such as baseline cognitive performance. In the particular example application studied, arithmetic performance was improved by applying different frequency-current tACS combinations over the left frontoparietal network.

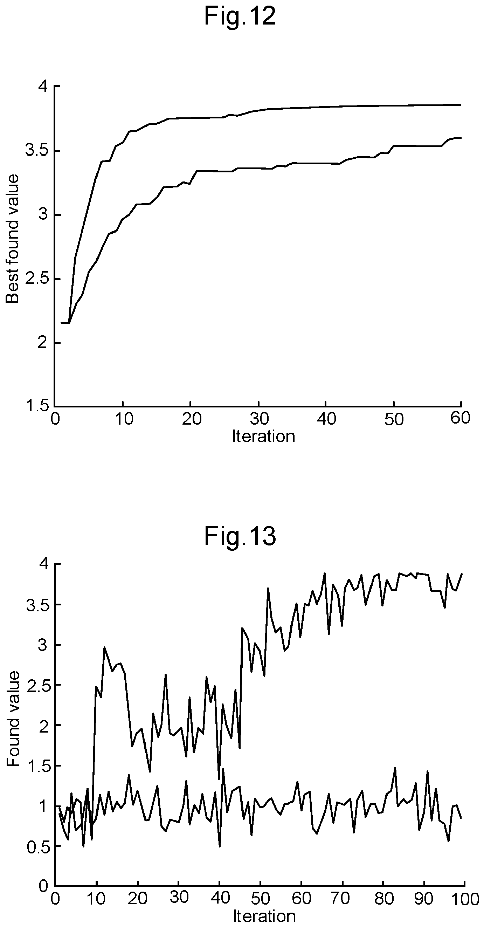

Figure 12 shows the results of running a simulation analysis (a Hartmann 3-Dimensional simulation - further details are provided below) to compare the performance of the present method for finding optimal parameters for transcranial stimulation (upper curve, based on using the EI acquisition function) with what can be obtained by randomly sampling the parameter space (lower curve). Over the 60 iteration window depicted, it can be seen that the best found value rises much more quickly using the present method than with random sampling and reaches a significantly higher level early in the iteration window than is ever reached by the random sampling approach.

Figure 13 compares use of a BO that only models parameters for stimulation (lower curve) with use of a personalized BO that jointly models parameters for stimulation and baseline cognitive performance (upper curve). It can be seen that the personalized BO (upper curve) is able to efficiently find the higher found values along 100 iterations while the conventional BO that only models the parameters for stimulation (lower curve) does not. In other words, higher cognitive performance levels (e.g., drift rate values) are attained when using the methodology of the present disclosure based on joint modelling of parameters for stimulation and baseline data, in comparison to random sampling of parameters. This finding applies regardless of whether EI or GP-UCB acquisition functions were used.

*EEG and baseline ability*

Previous literature suggests a positive relationship between frontoparietal theta (4-7.5 Hz) connectivity and arithmetic baseline ability due to the importance of this frequency band in high-level cognitive processing including arithmetic. However, none was found when running a regression model for connectivity in the 4-8 Hz range (all p>.3). The same applied to beta (13-40 Hz) connectivity (all p>.1) and frontal theta power (p=.88). However, our findings from the BO models highlights a strong performance effect in the beta frequency range (14-30 Hz) for subjects with average and high baseline abilities. We therefore examined the relationship between baseline ability and baseline beta power in an exploratory manner. We found a positive relationship between the two (non-parametric (spearman) correlation: $r_s$ = .29, $p$ = .03). In other words, subjects with higher arithmetic baseline abilities have higher beta power in comparison to subjects with lower arithmetic baseline skills which corroborates the group-level BO model results discussed above with reference to Figures 6-8.

FURTHER DETAILS ABOUT EXAMPLE METHODS

*Subjects and ethical permission*

Fifty subjects gave written consent before the start of the study. Each individual received a financial compensation of 50 pounds. In addition to this compensation, subjects had the chance of winning an additional 50 pounds based on their performance. Behavioural data from all 50 participants, aged between 18-30 years old, were used for the Bayesian Optimisation (mean age = 22.52 $\pm$ standard deviation (SD)=4.09, 31 females, all right-handed, finished education level; 1 GCSEs, 14 A-level, 17 Undergraduates and 18 Postgraduates; In the UK educational system GCSE refers to secondary education and A-level refers to advanced level that can lead to university). All subjects reported no counterindications to electrical stimulation (see supplementary data for full screening list) as well as no history of dyscalculia, dyslexia or attentional deficits. The proposed study received the ethical approval by The University of Oxford Medical Sciences Interdivisional Research Ethics Committee (protocol number: MSD-IDREC-C2-2014-033).

*Overview of experimental paradigm and stimuli*

Over the course of the experiment, participants completed four blocks of fifty multiplication problems, consisting of one baseline block (e.g., for obtaining a baseline metric value) and three stimulation blocks (e.g., for obtaining test data for refining the Gaussian process model). After an initial 4-minute resting-state EEG (rs-EEG) was recorded, the task was explained to the subjects and they completed 10 practice trials, followed by the baseline block during which no tACS was administered. Participants then underwent three blocks of multiplications in which they received tACS. Prior to each tACS block, the BO algorithm was run to determine the stimulation parameters (current intensity and frequency) to be delivered during the upcoming experimental block, based on the individual participant's performance in the baseline block (i.e. based on the baseline data value for that subject). The stimulation parameters were automatically selected by the algorithm and administered whilst maintaining blinding in both the participant and experimenter. Rs-EEG was again recorded after each stimulation block.

*Behavioural Stimuli*

Arithmetic performance was tested using an arithmetic calculation paradigm, consisting of problems involving a single-digit number multiplied by a two-digit number, with a three-digit outcome. A calculation paradigm was used instead of a retrieval paradigm since calculation has been associated with an increased activation in the frontoparietal network. None of the multiplications included operands with the digits 0, 1, 2, to account for variations in difficulty. In addition, the two-digit operand was not smaller than 15 and did not exist out of identical digits. Subjects were visually presented with a multiplication problem on a screen with a correct and incorrect answer positioned under the multiplication problem on the left and right side. The position of the correct and incorrect answer was randomly allocated to the right and left sides of the screen and they always differed by 10 digits. Lastly, every arithmetic multiplication problem consisted of a novel problem.

*Measurement of baseline abilities*

An arithmetic baseline task containing 50 different arithmetic multiplications was presented to measure individual arithmetic ability in terms of response times and accuracy. Subsequently, baseline drift rates were calculated for each subject according to the two-choice EZ-diffusion model. This model was chosen to reliably combine response time and accuracy in one outcome that can be optimised through the Bayesian Optimisation procedure. The 50 trials completed in the baseline block were randomly divided into two, and two separate drift rates were calculated. One was used as a measure of participant's baseline ability, whilst the other was used to normalise the drift rates calculated during the optimisation phase (e.g., during the experimental procedure of the Bayesian Optimisation). This was done to eliminate dependency between participant's baseline ability score and the normalised score in each stimulation block. To reduce fatigue, subjects had a break of 30s after every 10 trials. After completing the baseline task, subjects had a short break (~3 minutes) where after they continued with the experimental procedure of the Bayesian Optimisation.

*Experimental procedure of the Bayesian Optimisation*

Prior to each stimulation block, the Bayesian Optimisation procedure was run in order to determine the stimulation parameters to use. In total, 150 diverse multiplication problems (three blocks of 50 trials) were administered during the experimental procedure. The combination of tACS parameters (frequency and current) were changed for every subject and between every block depending on the Bayesian optimisation. In addition, performance from the baseline task was entered as a contextual variable (e.g., as the baseline metric value c). Thus, behavioural performance optimisation relied on the frequency and current of tACS together with the baseline cognitive ability, as indicated for example by the drift rate as a baseline metric value. Every subject received three different frequency- current tACS combinations whilst mentally calculating arithmetic problems. Subsequently, subjects had to indicate which answer was correct as fast as possible. Indication of the correct answer was done by pressing either a left or right button on a response box situated in front of the participant corresponding to the position of the answer on the screen. It was explained to the participant that they must answer as accurately and as quickly as possible. After every block, performance drift rates were calculated immediately, and another rs-EEG was measured for four minutes.

*Transcranial Alternating Current Stimulation (tACS)*

The alternating current stimulation was always administered to the left frontoparietal network. The tACS procedures included two stimulation (3.14 mm diameter) NG Pistim Ag/AgCl electrodes (F3 and P3) with one return electrode (Cz) using the Starstim 32 (Neuroelectrics, Barcelona). The impedances of the electrodes were held at <10 kQ. The stimulation intensity ranged between 0.1 mA - 1.6 mA peak-to- peak in steps of 0.1 for the burn-in phase of the study. For the optimisation, 0 mA was also added to control for possible sham influences. We chose this maximum stimulation intensity based on a small pilot study on 3 subjects to determine the maximum comfortable intensity.
Stimulation was administered in a double-blind manner during the three experimental blocks with a maximum of 10 minutes for each block. Stimulation started 45 s before the start of the block and changed after every block. If the subjects received a stimulation intensity of 0 mA during a block (sham stimulation), a ramp-up and a ramp-down of 30 s was initiated to provide the initial skin sensations during stimulation to ensure blinding. When the subject completed a block within 10 minutes, stimulation was ramped down for 30 s and the subject proceeded to the four-minute rs-EEG. Note that in cases where subjects completed the task faster than 10 minutes, they did not receive the full length of stimulation (24 of 150 stimulation blocks had a duration less than 10 minutes but more than 7.84 min and 126 stimulation blocks had a duration more than 10 minutes). This poses no problem since the current study only investigated the online effects of tACS. After completing a block related to one tACS combination the participant filled out a questionnaire in which they were asked several questions designed to gauge the level of sensations experienced during stimulation (see supplementary information for the full questionnaire). We used this data to assess the relationship between intensity rating of every sensation and tACS amplitude.

*Simulation analysis*

Several simulations were run to validate the cBO procedure during arithmetic performance and tACS. This analysis aimed to show that BO can outperform random sampling of different frequency-current tACS combinations. In addition, we wanted to compare the effects of the different acquisition functions (EI and UCB) and different dimensions ranging from 3 to 6. Note that the current study contained three dimensions, namely frequency, current, and individualised baseline ability (baseline metric value). Therefore, we ran 60 iterations using the Hartmann function that included four local minima for three dimensions similar to our experimental procedure of the BO in which we have three dimensions. The same was done for the Hartmann functions using 6 dimensions, the Ackley function using 5 dimensions, and the G-function using 4 dimensions. These simulations were noise free as BO is used mainly in noise free contexts. In contrast, human studies are prone to noisy evaluations. Therefore, we decided to introduce noise in the simulation by running the same Hartmann 3-Dimensional function for different noise variation values $(\sigma_n^2)$. Performance in these simulations were compared in terms of the distance from the known optimiser location with the Euclidean distance as metric. Lastly, we used the Hartmann function to compare the BO that models only parameters for stimulation with the BO that jointly models parameters for stimulation and baseline data.

*EEG analysis of spectral power and frontoparietal theta connectivity*

The rs-EEG data of the remaining datasets were separated in 2 second segments with an overlap of 1 second and windowed with a Hann window. Subsequently, data were transformed into the frequency domain via Fast Fourier Transformation (FFT). Theta (4-7.5 Hz) and beta (14-30 Hz) frequency bands were calculated according to their relative power ($\mu V^2$) and normalised by means of dividing the absolute frequency power of each frequency band by the average absolute power in the 1.5-30 Hz range. In addition, we decided to normalise the power by means of dividing the absolute frequency power of the applied tACS frequency value by the average absolute power in the 4-50 Hz range. Weighted phase lag index (wPLI) in the theta and beta range was computed to determine the phase lag synchronisation between the left frontal and parietal areas at baseline and after every tACS block. This computation was done for the complementary channels F3 and P3. Theta wPLI was calculated for 4-8 Hz in steps of 1 Hz and beta wPLI was calculated from 14 - 30 Hz in steps of 4 Hz. Furthermore, we normalised wPLI by calculating the wPLI at the specific tACS frequency divided by the baseline wPLI at the same frequency.
First, outliers were removed with Cook's distance before running statistical models. In order to focus on the relation between arithmetic baseline ability and spectral power, separate regression models were run with theta and beta power as dependent factor. Likewise, we tested if there was a relation between frontoparietal theta and beta connectivity scores by running several regression models in steps of 1 Hz for theta wPLI and steps of 4 Hz.

*Experimental work on ADHD*

The following describes work demonstrating the effectiveness of the disclosed method of obtaining personalized parameters for transcranial electrical stimulation (referred to below as "tES") when applied to treating Attention Deficit Hyperactivity Disorder (ADHD).

A simulated function was designed to demonstrate the utility of taking into account ADHD heterogeneity in order to tailor optimal tES parameters using personalized Bayesian optimization. The simulated function was set up with two input dimensions: (1) tES Current Intensity (mA) ranging from 0.1 to 2.0 and (2) Neurophysiology (TBR) ranging from 0.6 to 3.0. The Neurophysiology was considered as the personalized variable, which is specific to each participant. The output of the simulated function was the clinical outcome, which for illustrative purposes ranged from -1 (poor outcome) to 1 (desired outcome).

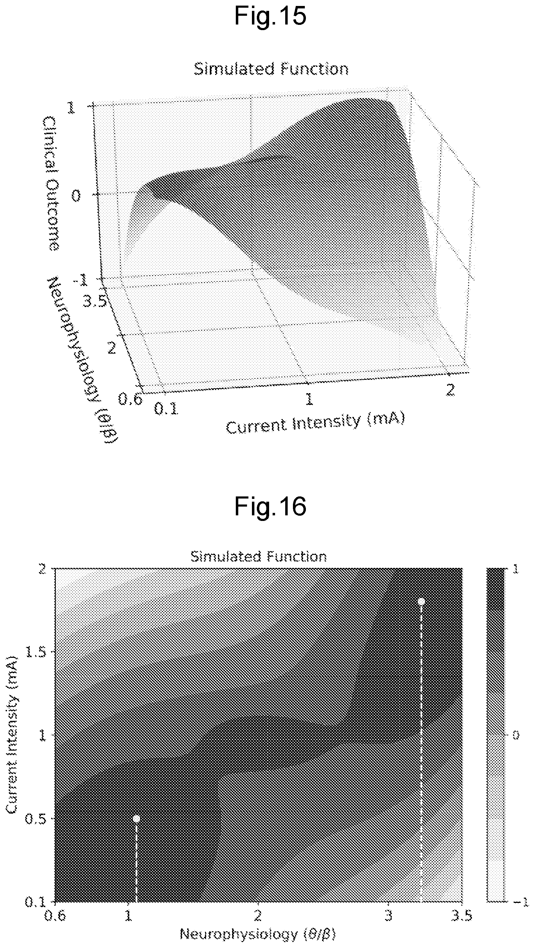

The simulated function is visualized in Figure 15 (in 3D) and 16 (in 2D, with peak points indicated by white circles). Based on previous findings, it was expected that those with higher TBR would require a higher current than those with lower TBR. Given the personalized variable of Neurophysiology, the inventors selected the Current Intensity, tested the function, and observed the Clinical Outcome. Note that it is neither possible to control nor optimize this personalized variable.

Three approaches were run and compared: 1) Random search, 2) non-personalized Bayesian optimization (hereafter, Bayesian optimization), and 3) personalized Bayesian optimization (which may be implemented using any of the methods for obtaining personalized parameters described above), over 30 iterations including 6 randomly chosen points at the beginning. The program was implemented in Python. Each iteration took two seconds to suggest a new parameter.

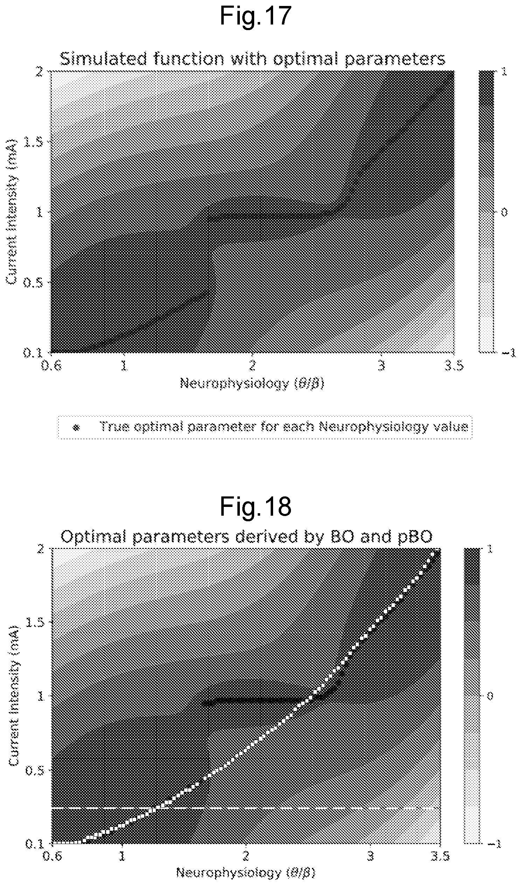

The true optimal parameter for each value of Neurophysiology, which is unknown to the scientist/clinician, are shown as black dots in Figure 17. It can be seen that different Neurophysiology values will require different Current Intensity to allow for the best Clinical Outcome.

The best parameter estimated at the final iteration by Bayesian optimization and personalized Bayesian optimization are visualized in Figure 18. The parameters suggested by personalized Bayesian optimization are depicted by white squares. The true optimal parameters are depicted by black dots. The results of Bayesian optimization are depicted by the white broken line. It can be seen that the parameters suggested by personalized Bayesian optimization (white squares) are very close to the true optimal parameters (black dots). In contrast, the Bayesian optimization, which does not consider the personalized Neurophysiology values, suggests a flat line (white broken line).

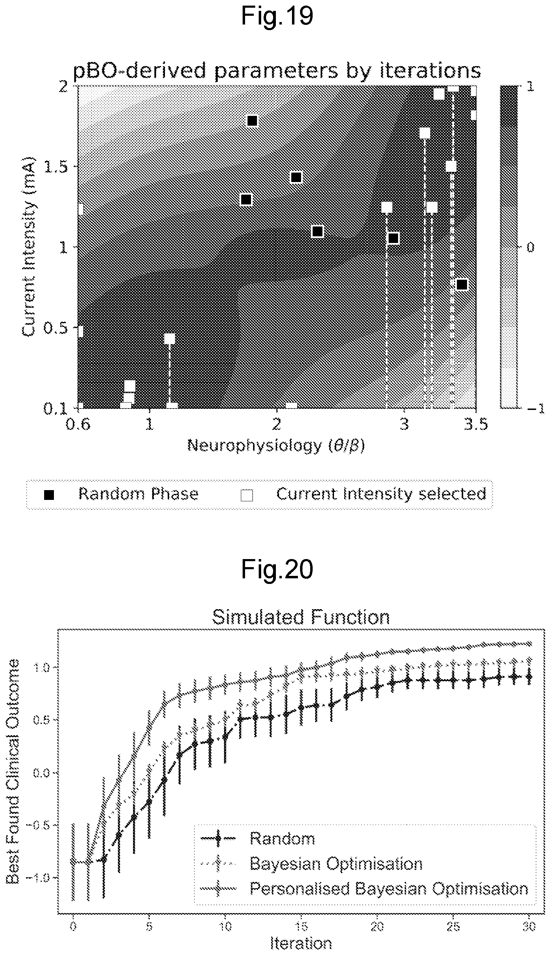

Figure 19 plots the parameters selected by personalized Bayesian optimization over iterations. The randomly chosen points from the initial phase are represented by black squares and the points selected by personalized Bayesian optimization are represented by white squares. These points are conditioned on the Neurophysiology value, as indicated by the white broken lines. From this it becomes evident that personalized Bayesian optimization can identify the high-performing region, as most of its selected parameters remain within the high value region (dark grey). One desirable property of personalized Bayesian optimization is that, while mainly selecting points in high value regions, it preserves some probability for exploration to gain information about the underlying function.

Figure 20 compares the performance across methods. Figure 20 shows that personalized Bayesian optimization outperformed both Bayesian optimization and Random Search in terms of providing the best clinical outcome. The performance of personalized Bayesian optimization at an early stage (at iteration 10) is achieved only later by Bayesian optimization and Random Search (after 20 and 30 iterations, respectively). In other words, personalized Bayesian optimization was two times faster than Bayesian optimization and three times faster than Random Search in this experiment, therefore requiring significantly less resources.

**Claims**

**1.** A computer-implemented method for obtaining personalized parameters for transcranial stimulation, comprising:

receiving baseline data about a test subject (S1), the baseline data comprising information about the test subject acquired prior to transcranial stimulation applied to the test subject; and
using a Gaussian process model of performance of one or more training subjects to obtain personalized parameters for transcranial stimulation for the test subject based on the received baseline data (S2), wherein:
the Gaussian process jointly models subject performance during and/or after transcranial stimulation as a function of both

i) parameters defining the transcranial stimulation; and
ii) baseline data for the one or more training subjects.

2. The method of claim 1, wherein the baseline data represents a performance without influence from transcranial stimulation.

3. The method of claim 1 or 2, wherein the obtaining of the personalized parameters (S2) further comprises:

   obtaining test data representing performances of the test subject during and/or after application of transcranial stimulation with multiple respective combinations of parameters;
   refining the Gaussian process model using the obtained test data; and
   using the refined Gaussian process model to obtain the personalized parameters.

4. The method of claim 3, wherein the Gaussian process model is refined in an iterative Bayesian optimization process that in each step chooses to sample next where a combination of parameters for stimulation optimizes an acquisition function.

5. The method of claim 4, wherein the acquisition function is configured to be computationally cheaper to optimize than an objective function of the Gaussian process model and wherein the acquisition function optionally comprises one or more of the following: the Gaussian process upper confidence bound; and the expected improvement.

6. The method of any preceding claim, further comprising training a Gaussian process model to provide the Gaussian process model used to obtain the personalized parameters.

7. The method of claim 6, wherein the training of the Gaussian process model comprises using training data representing performances of each of multiple training subjects during and/or after application of transcranial stimulation with multiple respective combinations of parameters for each training subject.

8. The method of claim 7, wherein the Gaussian process model is trained in an iterative Bayesian optimization process that in each step chooses to sample next where a combination of parameters for stimulation optimizes an acquisition function.

9. The method of claim 8, wherein the acquisition function is configured to be computationally cheaper to optimize than an objective function of the Gaussian process model and wherein the acquisition function optionally comprises one or more of the following: the Gaussian process upper confidence bound; and the expected improvement.

10. The method of any preceding claim, wherein:

    the transcranial stimulation comprises any one or more of the following: transcranial alternating current stimulation; transcranial random noise stimulation; transcranial direct current stimulation; transcranial magnetic stimulation; transcranial focused ultrasound; and transcranial light stimulation; and/or
    the parameters for transcranial stimulation comprise one or more of the following: frequency, frequencies, current, phase, duration, dose, and brain region.

11. The method of any preceding claim, wherein:

    the performances are represented by one or more of the following: accuracy; reaction time; test score; and subjective reporting; and/or
    the performances are represented by a drift rate value obtained by applying a diffusion decision model to the results of tests performed by the respective subject.

12. A data processing apparatus comprising a processor configured to perform the method of any preceding claim.

13. A computer program comprising instructions which, when the program is executed by a computer, cause the computer to carry out the method of any of claims 1-11.

14. A computer-readable data carrier having stored thereon the computer program of claim 13.

15. A transcranial stimulation system, comprising:

    a stimulation unit configured to provide transcranial stimulation to a subject; and

a data processing apparatus configured to control operating parameters of the stimulation unit to define parameters of the transcranial stimulation, wherein:

the data processing apparatus is configured to perform the method of any of claims 1-11 to obtain personalized parameters for the subject and control the operating parameters of the stimulation unit based on the obtained personalized parameters.

**Patentansprüche**

1. Computerimplementiertes Verfahren zum Erlangen personalisierter Parameter für transkranielle Stimulation, umfassend:

   Empfangen von Ausgangsdaten über ein Testsubjekt (S1), die Ausgangsdaten umfassend Informationen über das Testsubjekt, die vor Anwenden der transkraniellen Stimulation an dem Testsubjekt erfasst werden; und
   Verwenden eines Gaußschen Prozessmodells einer Leistung eines oder mehrerer Trainingssubjekte, um personalisierte Parameter für transkranielle Stimulation für das Testsubjekt basierend auf den empfangenen Ausgangsdaten (S2) zu erlangen, wobei:
   der Gaußsche Prozess gemeinsam die Leistung eines Subjekts während und/oder nach transkranieller Stimulation abhängig von folgenden beiden modelliert

   i) Parametern, die die transkranielle Stimulation definieren; und
   ii) Ausgangsdaten für das eine oder die mehreren Trainingssubjekte.

2. Verfahren nach Anspruch 1, wobei die Ausgangsdaten eine Leistung ohne Einfluss von transkranieller Stimulation darstellen.

3. Verfahren nach Anspruch 1 oder 2, wobei das Erlangen der personalisierten Parameter (S2) ferner Folgendes umfasst:

   Erlangen von Testdaten, die Leistungen des Testsubjekts während und/oder nach Anwenden von transkraniellen Stimulation mit mehreren entsprechenden Parameterkombinationen darstellen;
   Verfeinern des Gaußschen Prozessmodells unter Verwendung der erlangten Testdaten; und
   Verwenden des verfeinerten Gaußschen Prozessmodells, um die personalisierten Parameter zu erlangen.

4. Verfahren nach Anspruch 3, wobei das Gaußsche Prozessmodell in einem iterativen Bayesschen Optimierungsprozess verfeinert wird, der in j edem Schritt die nächste Probe auswählt, bei der eine Kombination von Parametern zur Stimulation eine Erfassungsfunktion optimiert.

5. Verfahren nach Anspruch 4, wobei die Erfassungsfunktion so konfiguriert ist, dass sie rechnerisch billiger zu optimieren ist als eine Zielfunktion des Gaußschen Prozessmodells, und wobei die Erfassungsfunktion optional eines oder mehrere von Folgenden umfasst: die obere Konfidenzgrenze des Gaußschen Prozesses; und die erwartete Verbesserung.

6. Verfahren nach einem vorherigen Anspruch, ferner umfassend ein Trainieren eines Gaußschen Prozessmodells, um das Gaußsche Prozessmodell bereitzustellen, das verwendet wird, um die personalisierten Parameter zu erlangen.

7. Verfahren nach Anspruch 6, wobei das Training des Gaußschen Prozessmodells ein Verwenden von Trainingsdaten umfasst, die Leistungen jedes von mehreren Trainingssubjekten während und/oder nach Anwenden von transkranieller Stimulation mit mehreren jeweiligen Kombinationen von Parametern für jedes Trainingssubjekt darstellen.

8. Verfahren nach Anspruch 7, wobei das Gaußsche Prozessmodell in einem iterativen Bayesschen Optimierungsprozess trainiert wird, der in jedem Schritt die nächste Probe auswählt, bei der eine Kombination von Parametern zur Stimulation eine Erfassungsfunktion optimiert.

9. Verfahren nach Anspruch 8, wobei die Erfassungsfunktion so konfiguriert ist, dass sie rechnerisch billiger zu optimieren ist als eine Zielfunktion des Gaußschen Prozessmodells, und wobei die Erfassungsfunktion optional eines oder mehrere von Folgenden umfasst: die obere Konfidenzgrenze des Gaußschen Prozesses; und die erwartete Verbesserung.

10. Verfahren nach einem vorherigen Anspruch, wobei:

die transkranielle Stimulation eine oder mehrere der Folgenden umfasst: transkranielle Wechselstromstimulation; transkranielle Zufallsrauschstimulation; transkranielle Gleichstromstimulation; transkranielle Magnetstimulation; transkranieller fokussierter Ultraschall; und transkranielle Lichtstimulation; und/oder
die Parameter für transkranielle Stimulation umfassen eines oder mehrere von Folgenden: Frequenz, Frequenzen, Stromstärke, Phase, Dauer, Dosis und Gehirnregion.

11. Verfahren nach einem vorherigen Anspruch, wobei:

die Leistungen durch eines oder mehrere von Folgenden dargestellt sind: Genauigkeit; Reaktionszeit; Testergebnis; und subjektive Berichterstattung; und/oder
die Leistungen durch einen Driftratenwert dargestellt sind, der durch Anwenden eines Diffusionsentscheidungsmodells auf die Ergebnisse der von der jeweiligen Testsubjekt durchgeführten Tests erlangt wird.

12. Datenverarbeitungsgerät, umfassend einen Prozessor, der konfiguriert ist, um das Verfahren nach einem vorherigen Anspruch durchzuführen.

13. Computerprogramm, umfassend Anweisungen, die, wenn das Programm von einem Computer ausgeführt wird, den Computer veranlassen, das Verfahren nach einem der Ansprüche 1-11 durchzuführen.

14. Computerlesbarer Datenträger, auf dem das Computerprogramm nach Anspruch 13 gespeichert ist.

15. System für transkranielle Stimulation, umfassend:

eine Stimulationseinheit, die konfiguriert ist, um transkranielle Stimulation für ein Subjekt bereitzustellen; und
ein Datenverarbeitungsgerät, das konfiguriert ist, um Betriebsparameter der Stimulationseinheit zu steuern, um Parameter der transkraniellen Stimulation zu definieren, wobei:
die Datenverarbeitungsgerät konfiguriert ist, um das Verfahren nach einem der Ansprüche 1-11 durchzuführen, um personalisierte Parameter für das Subjekt zu erlangen und die Betriebsparameter der Stimulationseinheit basierend auf den Erlangten personalisierten Parameter zu steuern.

**Revendications**

1. Procédé mis en oeuvre par ordinateur pour obtenir des paramètres personnalisés pour une stimulation transcrânienne, comprenant :

la réception de données de base concernant un sujet de test (S1), les données de base comprenant des informations sur le sujet de test acquises avant la stimulation transcrânienne appliquée au sujet de test ; et
l'utilisation d'un modèle de processus gaussien de performance d'un ou plusieurs sujets d'apprentissage pour obtenir des paramètres personnalisés pour la stimulation transcrânienne pour le sujet de test sur la base des données de base reçues (S2), dans lequel :
le processus gaussien modélise conjointement les performances du sujet pendant et/ou après la stimulation transcrânienne en fonction à la fois de

i) paramètres définissant la stimulation transcrânienne ; et
ii) données de base pour l'un ou plusieurs sujets d'apprentissage.

2. Procédé selon la revendication 1, dans lequel les données de base représentent une performance sans influence de la stimulation transcrânienne.

3. Procédé selon la revendication 1 ou 2, dans lequel l'obtention des paramètres personnalisés (S2) comprend en outre :

l'obtention de données de test représentant les performances du sujet de test pendant et/ou après l'application d'une stimulation transcrânienne avec de multiples combinaisons respectives de paramètres ;

l'affinage du modèle de processus gaussien en utilisant les données de test obtenues ; et
l'utilisation du modèle de processus gaussien affiné pour obtenir les paramètres personnalisés.

4. Procédé selon la revendication 3, dans lequel le modèle de processus gaussien est affiné dans un processus d'optimisation bayésien itératif qui, à chaque étape, choisit d'échantillonner ensuite où une combinaison de paramètres de stimulation optimise une fonction d'acquisition.

5. Procédé selon la revendication 4, dans lequel la fonction d'acquisition est configurée pour être informatiquement moins coûteuse à optimiser qu'une fonction objective du modèle de processus gaussien et dans lequel la fonction d'acquisition comprend facultativement un ou plusieurs des éléments suivants : la limite de confiance supérieure du processus gaussien ; et l'amélioration attendue.

6. Procédé selon l'une quelconque des revendications précédentes, comprenant en outre l'apprentissage d'un modèle de processus gaussien pour fournir le modèle de processus gaussien utilisé pour obtenir les paramètres personnalisés.

7. Procédé selon la revendication 6, dans lequel l'apprentissage du modèle de processus gaussien comprend l'utilisation de données d'apprentissage représentant les performances de chacun des multiples sujets d'apprentissage pendant et/ou après l'application d'une stimulation transcrânienne avec de multiples combinaisons respectives de paramètres pour chaque sujet d'apprentissage.

8. Procédé selon la revendication 7, dans lequel le modèle de processus gaussien est formé dans un processus d'optimisation bayésien itératif qui, à chaque étape, choisit d'échantillonner ensuite où une combinaison de paramètres de stimulation optimise une fonction d'acquisition.

9. Procédé selon la revendication 8, dans lequel la fonction d'acquisition est configurée pour être moins coûteuse à optimiser en calcul qu'une fonction objective du modèle de processus gaussien et dans lequel la fonction d'acquisition comprend facultativement un ou plusieurs des éléments suivants : la limite de confiance supérieure du processus gaussien ; et l'amélioration attendue.

10. Procédé selon une quelconque revendication précédente, dans lequel :

la stimulation transcrânienne comprend l'un quelconque ou plusieurs des éléments suivants : stimulation transcrânienne par courant alternatif ; stimulation transcrânienne par bruit aléatoire ; stimulation transcrânienne par courant continu ; stimulation transcrânienne magnétique ; ultrasons focalisés transcrâniens ; et stimulation lumineuse transcrânienne ; et/ou
les paramètres de stimulation transcrânienne comprennent un ou plusieurs des éléments suivants : fréquence, fréquences, courant, phase, durée, dose et région cérébrale.

11. Procédé selon une quelconque revendication précédente, dans lequel :

les performances sont représentées par un ou plusieurs des éléments suivants : précision ; temps de réaction; score du test ; et rapport subjectif ; et/ou
les performances sont représentées par une valeur de vitesse de dérive obtenue en appliquant un modèle de décision de diffusion aux résultats de tests effectués par le sujet respectif.

12. Appareil de traitement de données comprenant un processeur configuré pour exécuter le procédé selon l'une quelconque des revendications précédentes.

13. Programme informatique comprenant des instructions qui, lorsque le programme est exécuté par un ordinateur, amènent l'ordinateur à effectuer le procédé selon l'une quelconque des revendications 1-11.

14. Support lisible par ordinateur sur lequel est stocké le programme informatique selon la revendication 13.

15. Système de stimulation transcrânienne, comprenant :

une unité de stimulation configurée pour fournir une stimulation transcrânienne à un sujet ; et
un appareil de traitement de données configuré pour contrôler les paramètres de fonctionnement de l'unité de stimulation afin de définir les paramètres de la stimulation transcrânienne, dans lequel :
l'appareil de traitement de données est configuré pour exécuter le procédé selon l'une quelconque des revendications 1-11 pour obtenir des paramètres personnalisés pour le sujet et contrôler les paramètres de fonctionnement de l'unité de stimulation sur la base des paramètres personnalisés obtenus.

# Fig.1

S1 — Receive baseline data ← Baseline data

S21 — Use model to obtain parameters ← Pretrained model

S23 — Adjust model

Parameters

S2

S22 — Apply stimulation

Personalized parameters

Fig.2

Fig.3

Fig.4

Fig.5

Fig.6

**Mean - 1sd**

## Fig.7

Mean

Fig.8

**Mean + 1sd**

## Fig.9

Baseline Cognitive Performance

## Fig.10

Baseline Cognitive Performance

## Fig.11

# Fig.12

# Fig.13

# Fig.14

# Fig.15

Simulated Function

# Fig.16

Simulated Function

## Fig.17

Simulated function with optimal parameters

* True optimal parameter for each Neurophysiology value

## Fig.18

Optimal parameters derived by BO and pBO

## Fig.19

### pBO-derived parameters by iterations

■ Random Phase     □ Current Intensity selected

## Fig.20

### Simulated Function

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20190321583 A

**Non-patent literature cited in the description**

- **SRINIVAS, NIRANJAN ; ANDREAS KRAUSE ; SHAM KAKADE ; MATTHIAS SEEGER.** Gaussian process optimization in the bandit setting: no regret and experimental design. *Proceedings of the 27th International Conference on International Conference on Machine Learning,* 2010, 1015-1022